(19) Europäisches Patentamt
European Patent Office
Office européen des brevets

(11) Veröffentlichungsnummer : **0 234 004 B1**

# (12) EUROPÄISCHE PATENTSCHRIFT

(45) Veröffentlichungstag der Patentschrift : **23.01.91 Patentblatt 91/04**

(51) Int. Cl.$^5$ : **A61K 9/22**, A61L 27/00, A61K 31/435, A61K 31/47, A61K 31/50, A61K 31/505

(21) Anmeldenummer : **86116207.1**

(22) Anmeldetag : **22.11.86**

## (54) Implantierbares Pharmakadepot mit Gyrasehemmern.

Die Akte enthält technische Angaben, die nach dem Eingang der Anmeldung eingereicht wurden und die nicht in dieser Patentschrift enthalten sind.

(30) Priorität : **05.12.85 DE 3542972**

(43) Veröffentlichungstag der Anmeldung : **02.09.87 Patentblatt 87/36**

(45) Bekanntmachung des Hinweises auf die Patenterteilung : **23.01.91 Patentblatt 91/04**

(84) Benannte Vertragsstaaten : **AT BE CH DE ES FR GB IT LI NL SE**

(56) Entgegenhaltungen :
**EP-A- 0 025 698**
**US-A- 4 563 485**

(73) Patentinhaber : **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder : **Wahlig, Helmut, Dr.**
**Roemheldweg 16**
**D-6100 Darmstadt (DE)**
Erfinder : **Dingeldein, Elvira, Dr.**
**Am Spitzenpfad 9**
**D-6072 Dreieich (DE)**
Erfinder : **Rothe, Johannes, Dr.**
**Am Hang 11**
**D-6101 Rossdorf 1 (DE)**
Erfinder : **Stille, Wolfgang, Prof. Dr.**
**Töplitzstrasse 7**
**D-6000 Frankfurt a. M. (DE)**

**EP 0 234 004 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Gegenstand der Erfindung ist ein implantierbares Pharmakadepot, im wesentlichen bestehend aus physiologisch verträglichen Trägermaterialien der Gruppe und mindestens einem verzögert freizusetzenden Wirkstoff, dadurch gekennzeichnet, daß es als Wirkstoff ein Chemotherapeutikum des Gyrasehemmertyps enthält.

Polyacrylate und/oder Polymethacrylate, Tricalciumphosphat, Hydroxylapatit, Kollagen, Agarose, Gelatine und oder Fibrin,

Pharmakadepots, die diese Trägermaterialien und als Wirkstoffe bevorzugt Antibiotika enthalten, sind z.B. in den DE-OSen 23 20 373, 26 51 441, 26 57 370, 28 43 963, 29 35 194, 32 06 725, 32 06 726 und 33 34 595 sowie in der dort zitierten Literatur beschrieben. Wegen Einzelheiten der Herstellung und der Zusammensetzung der Pharmakadepots wird ausdrücklich auf diesen Stand der Technik verwiesen. Der hier gebrauchte Ausdruck "Pharmakadepot" ist in weitem Sinne zu verstehen ; er umschließt auch mit Gyrasehemmern versehene Implantate, z.B. künstliche Organe, Knochen- und Zahnteile, Zahnfüllungen, Gelenke, Knochennägel und -schrauben, Osteosyntheseplatten, Katheter, Sonden, Schrittmachergehäuse. Dabei können die Wirkstoffe auch in Form von Lacken (Filmen), Überzügen oder Gelen auf die Oberfläche dieser Gegenstände aufgebracht sein, z.B. durch Tauchen oder Besprühen. Auch gyrasehemmerhaltige Okklusivfolien sind in dem Ausdruck "Pharmakadepot" eingeschlossen.

Chemotherapeutika vom Typ der Gyrasehemmer ("Quinolones") sind in großer Zahl bekannt. Im Prinzip sind alle Gyrasehemmer für den erfindungsgemäßen Zweck geeignet.

Bevorzugte Gyrasehemmer entsprechen der Formel I

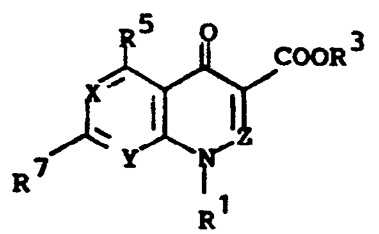

I

worin
X N oder $CR^6$,
Y N oder $Cr^8$,
Z N oder CH,
$R^1$ Alkyl, Alkenyl, Hydroxyalkyl, Hal-alkyl, $NR^9R^{10}$, $OR^{11}$, cyclo-alkyl, Ar oder Ar-alkyl,
$R^3$ H oder Alkyl,
$R^5$ H, Hal, Alkyl oder Alkoxy,
$R^6$ H, Hal, Alkyl, Alkoxy oder $NO_2$,
$R^7$ Alkyl, Hal, $NR^9R^{10}$ oder einen 5- oder 6-gliedrigen N-haltigen gesättigten oder ungesättigten heterocyclischen Rest, der auch ein weiteres Heteroatom enthalten und/oder substituiert sein kann,
$R^6$ und $R^7$ zusammen auch Methylendioxy,
$R^8$ H, Hal, Alkyl oder Alkoxy,
$R^8$ und $R^1$ zusammen auch $-O-CH_2-CHAlkyl-$ oder $-CH_2CH_2-CHAlkyl-$,
$R^9$, $R^{10}$ und $R^{11}$ jeweils H oder Alkyl,
Ar eine unsubstituierte oder substituierte Phenylgruppe und
Hal F, Cl, Br oder J bedeuten und
die Alkyl-, Alkenyl- und Alkoxygruppen jeweils bis zu 6 C-Atome enthalten.

Insbesondere sind Verbindungen der Formeln Ia bis Ic bevorzugt, die der Formel I entsprechen, worin jedoch in Ia Y N oder $CR^8$
Z N oder CH,
$R^1$ Alkyl, Hal-alkyl, NH-Alkyl, O-Alkyl, Cyclo-alkyl oder Hal-phenyl,
$R^3$ H,
$R^5$ H,
$R^6$ H oder Hal,
$R^7$ Alkyl, Hal oder einen 5- oder 6-gliedrigen N-haltigen gesättigten oder ungesättigten heterocyclischen Rest, der ein weiteres N-Atom enthalten und/oder durch ein bis zwei Alkyl-, Amino- oder Alkylaminoalkylgruppe(n) substituiert sein kann,

$R^6$ und $R^7$ zusammen auch Methylendioxy,
$R^8$ H, Hal oder Alkyl,
$R^8$ und $R^1$ zusammen auch -O-$CH_2$-CHAlkyl- oder -$CH_2CH_2$-CHAlkyl- und
Hal F oder Cl bedeuten und
die Alkylgruppen jeweils 1-3 C-Atome enthalten ;
in Ib Y N oder $CR^8$,
Z N oder CH,
$R^1$ Alkyl, 2-Fluorethyl, Methylamino, Methoxy, Cyclopropyl oder p-Fluor-phenyl,
$R^3$ und $R^5$ jeweils H,
$R^6$ H oder F,
$R^7$ Methyl, Cl, Pyrryl, Pyrrolidino, 3,4-Dimethylpyrrolidino, 3-Aminopyrrolidino, 3-Amino-4-methylpyrrolidino, 3-Ethylaminomethyl-pyrrolidino, Pyridyl, Piperidyl, Piperazino oder 3- oder 4-Methylpiperazino,
$R^6$ und $R^7$ zusammen auch Methylendioxy,
$R^8$ H, Methyl oder F und
$R^8$ und $R^1$ zusammen auch -$OCH_2$-$CH(CH_3)$- oder -$CH_2CH_2$-$CH(CH_3)$- bedeuten und die Alkylgruppen jeweils 1-3 C-Atome enthalten,
in Ic Y N oder $CR^8$,
Z N oder CH,
$R^1$ Ethyl oder Cyclopropyl,
$R^3$ und $R^5$ jeweils H,
$R^6$ F,
$R^7$ Piperazino oder 3- oder 4-Methyl-piperazino,
$R^8$ H, und
$R^8$ und $R^1$ zusammen auch -O-$CH_2$-$CH(CH_3)$- bedeuten.

Bevorzugte Gyrasehemmer sind im einzelnen Ciprofloxacin und Ofloxacin, weiterhin Amifloxacin, Cinoxacin, Difloxacin (A-56619), Enoxacin, Flumequin, Irloxacin, Miloxacin, Nalidixinsäure, Norfloxacin, Oxolinsäure, Pefloxacin, Pipemidsäure, Piromidsäure, Rosoxacin, A-56620, A-57132, A-60969, A-656619, A-656620, AM-833, AT-3295, AT-3765, CI-934, E 3432, E-3499, EN-272, NY-198, OPC-7594, Ro 23-6240, S-25930, S-25932, T-14097, 79286-76-3, 80-1057118, ferner 1-Ethyl-2-phenyl-4-pyridon-5-carbonsäure und deren im Phenylring substituierte Derivate.

Die Pharmakadepots enthalten vorzugsweise 0,01 bis 10, insbesondere 0,1 bis 3, vor allem 0,1 bis 1% des Chemotherapeutikums.

Pharmakadepots auf Basis von Polyacrylaten und/oder Polymethacrylaten sind besonders bevorzugt. Sie können z.B. in Analogie zu den Angaben in den DE-OSen 23 20 373 und 26 51 441 hergestellt werden, indem man etwa 50 bis 75 Gewichtsteile eines feinteiligen Polymerisats von Acryl- und/oder Methacrylsäureestern, das gegebenenfalls weitere Zusätze, z.B. Röntgenkontrastmittel wie $ZrO_2$ Farbstoffe wie Chlorophyll und/oder Katalysatoren enthalten kann, 0,01 bis 10 Gewichtsteile des Chemotherapeutikums und 20 bis 45 Gewichtsteile eines Acryl- und/oder Methacrylsäureesters, der gegebenenfalls weitere Zusätze, z.B. Stabilisatoren und/oder Polymerisationsbeschleuniger enthält, zu einer halbfesten Paste vermengt, diese in eine gewünschte Form bringt und durch Polymerisation und Vernetzung aushärten läßt. Das Gemisch kann gewünschtenfalls zusätzlich eine oder mehrere physiologisch unbedenkliche Aminosäuren enthalten, vorzugsweise in einer Menge von 1 bis 15 Gewichtsteilen. Ein zusätzlicher Gehalt an vorzugsweise natürlichen Aminosäuren, z.B. Glycin, Alanin, Leucin, Threonin, Valin, Serin, Hydroxyprolin, Prolin, Histidin und/oder Arginin, kann die zuverlässige Freisetzung des Wirkstoffs fördern.

Die Kunststoffmaterialien auf Basis von Polyacrylaten und/oder Polymethacrylaten, die als Ausgangsmaterialien eingesetzt werden können, sind an sich bekannt. Sehr gebräuchlich ist z.B. ein Knochenzement, der in einer Normalpackung 2 Beutel mit je etwa 40 g Pulver und 2 Ampullen mit je 20 ml Flüssigkeit enthält. Das Pulver ist ein feines Perlpolymerisat aus Methacrylsäuremethylester mit einem Copolymeranteil von Acrylsäuremethylester. Als Katalysator sind dem Pulver etwa 0,5% Dibenzoylperoxid zugesetzt. Zur Kennzeichnung des Materials sind bei der Herstellung Spuren von Chlorophyll mit einpolymerisiert. Als Röntgenkontrastmittel kann das Pulver gegebenenfalls zusätzlich z.B. Zirkondioxid enthalten. Die zugehörige Flüssigkeit besteht aus monomerem Methacrylsäuremethylester, dem als Polymerisationsbeschleuniger etwa 0,7% Dimethyl-p-toluidin sowie als Stabilisator Spuren von Hydrochinon zugesetzt sind. Auch diese Flüssigkeit ist in der Regel zur Kennzeichnung mit Spuren von Chlorophyll eingefärbt. Das in Polyethylenbeutel abgepackte Pulver ist mit Ethylenoxid sterilisiert. Die Flüssigkeit ist steril filtriert und in Glasampullen abgefüllt.

Beim Zusammenmischen von zwei Gewichtsteilen Pulver mit einem Gewichtsteil Flüssigkeit reagiert das Dibenzoylperoxid mit dem Dimethyl-p-toluidin in der Flüssigkeit, wodurch die radikalische Polymerisation angeregt wird. Die Mischung ist so abgestimmt, daß sie schon nach etwa einer Minute als teigige Paste verwendet werden kann. Diese Paste bleibt für mehrere Minuten knetbar und beginnt dann unter Wärmeentwicklung auszuhärten. Nach etwa 5 bis 10 Minuten ist die Polymerisation im wesentlichen abgeschlossen. Während der Polymerisation, solange die Paste noch formbar ist, kann diese in jede gewünschte Form gebracht werden, also z.B. zum Ausfüllen von Knochenhöhlen oder zum Einzementieren von Prothesen direkt in den Körper gebracht oder zur Herstellung von Formkörpern verwendet werden, die extrakorporal aushärten und danach an beliebigen Körperstellen eingesetzt werden können.

Das Chemotherapeutikum und/oder die Aminosäure kann als feines Pulver den übrigen Bestandteilen zugemischt und so in dem entstehenden Polymeren homogen verteilt werden. Es kann aber auch bereits bei der Herstellung des Präpolymerisats in dieses eingearbeitet werden.

Das erfindungsgemäße Pharmakadepot kann fertig auspolymerisiert und damit in vorbestimmter Form zur Verfügung gestellt werden. Dies ist insbesondere dann der Fall, wenn z.B. beim Einsatz in Weichteilen lediglich die Funktion als lokale Wirkstoffquelle erfüllt werden muß. Dazu kann das Depot in beliebiger Form, z.B. als Granulat, als Kubus, Kugel oder Ellipsoid, als Folie, Platte, Stift, Röhre, Faser, Faden, Netz, Vlies oder anderer dem jeweiligen Einsatz angepaßter Form hergestellt werden.

Man kann aber auch dem Chirurgen das erfindungsgemäße Material als Vorprodukt in die Hand geben, so daß die Formgebung erst bei der Implantation erfolgt und somit das Pharmakadepot den örtlichen Gegebenheiten optimal angepaßt und das Material auch bei der Implantation von Prothesen wie ein herkömmlicher Knochenzement eingesetzt werden kann. Dazu können die Bestandteile analog den oben beschriebenen bekannten Knochenzementen gebrauchsfertig so abgepackt werden, daß aufeinander abgestimmte Mengen der Feststoffe und der Flüssigkeit (des Monomeren) in einer Packung vorliegen. Aus diesem Vorprodukt kann man dann in einfacher Weise das Pharmakadepot herstellen, indem man die Komponenten vermischt ; dabei wird durch den beigefügten Katalysator die Polymerisation des Monomeren in Gang gesetzt, und nach wenigen Minuten Reaktionszeit liegt das ausgehärtete Endprodukt vor. In der dazwischenliegenden Zeit, in der das Material plastisch verformbar ist, kann es in den Körper eingebracht und dabei geformt werden.

Pharmakadepots auf Basis Tricalciumphosphat können z.B. gemäß DE-OS 28 07 132 oder DE-OS 32 06 726 hergestellt werden. So kann pulverförmiges Tricalciumphosphat mit dem Chemotherapeutikum entweder direkt oder unter Zusatz von etwa 1-20% eines physiologisch unbedenklichen, vorzugsweise m Körper resorbierbaren Bindemittels, z.B. eines Kollagenabbauprodukts, Elastin oder bevorzugt Calciumsulfat, zu einem Pharmakadepot gepreßt werden, das nach der Implantation seine Form zunächst behält und den Wirkstoff protrahiert freisetzt, auf lange Sicht jedoch im Körper resorbiert wird. Man kann aber auch Granulate aus Tricalciumphosphat, vorzugsweise solche mit Korngrößen zwischen 0,1 und 8 mm, vorzugsweise zwischen 1,4 und 2,8 mm und Porenvolumina zwischen 40 und 90, vorzugsweise 60 und 80%, mit den Chemotherapeutika beschicken.

"Tricalciumphosphat" im Sinne der vorliegenden Erfindung umschließt nicht nur reine Tricalciumphosphate wie α-oder ß-Whitlockit, sondern auch Apatite, Hydroxylapatite (Durapatite) oder Phosphorit und ähnliche Stoffe, die nur annähernd durch die Formeln $Ca_3(PO_4)_2$ oder $Ca_5(PO_4)_3OH$ beschrieben werden können. Tricalciumphosphat soll auf jeden Fall im Körper resorbierbar sein und wird vorzugsweise in Pulverform eingesetzt, zweckmäßig mit Teilchengrößen von etwa 0,5 bis etwa 50 µm, vorzugsweise von etwa 0,5 bis etwa 20 µm.

Pharmakadepots auf Basis Tricalciumphosphat können z.B. durch Verpressen der Bestandteile bei Drucken von etwa 5 bis etwa 1200, vorzugsweise 100 bis 800 bar, und Temperaturen zwischen etwa 20 und etwa 150° hergestellt werden, und zwar in beliebigen, z.B. den oben angegebenen Formen.

Pharmakadepots auf Basis Kollagen können z.B. in Analogie zu den Angaben in den DE-OSen 28 43 963 oder 33 34 595 hergestellt werden.

Bevorzugt sind Kollagene, in denen die Porenstruktur des natürlichen Materials erhalten ist und die Porenvolumina von etwa 60 bis etwa 95% besitzen. Weiterhin sind rekonstituierte Kollagene mit Vlies- oder Schwammstruktur bevorzugt, insbesondere solche, die aus Fasern mit einer Länge von maximal 10 mm bestehen, und/oder in denen die Polypeptidketten zusätzlich vernetzt sind, z.B. mit Formaldehyd, Glutardialdehyd, Glyoxal oder Hexamethylendiisocyanat.

Depots auf Basis Kollagen enthalten zweckmäßig zusätzlich bioresorbierbare Bindemittel wir Glykolid-Lactid-Copolymere und/oder bioresorbierbare Polymere, z.B. saure Polysaccharide wir Pektinsäuren oder Alginsäuren und/oder deren Salze und/oder Calciumphosphat. Sie sind herstellbar durch Verpressen der Bestandteile bei Temperaturen zwischen etwa 20 und etwa 200°, vorzugsweise zwischen 70 und 150°, und Drucken zwischen etwa 300 und etwa 1200, vorzugsweise zwischen 500 und 800 bar. Depots auf Basis eines Kollagens mit erhaltener Porenstruktur sind z.B. erhältlich durch Tränken der Kollagen-Matrix mit einer Lösung eines Salzes des Chemotherapeutikums und eines Salzes des sauren Polysaccharids, und

anschließende pH-Verschiebung in einen Bereich von pH 4 bis 8. Depots auf Basis eines rekonstituierten Kollagens mit weitgehend erhaltener nativer Molekülstruktur können z.B. hergestellt werden, indem man zu einer Suspension eines solchen Kollagens das Chemotherapeutikum und ein saures Polysaccharid hinzugibt, wobei man vor oder nach der Zugabe den pH-Wert der Suspension auf einen Wert unterhalb 7 einstellt und die Suspension anschließend, gegebenenfalls nach Eingießen in Formen, gefriertrocknet. Mann kann auch aus rekonstituierten Kollagenfasern zunächst vliesartige oder schwammig-poröse Formkörper herstellen, die man anschließend mit einer Lösung des Chemotherapeutikums und des Polysaccharids tränkt, dann den pH-Wert der Tauchlösung auf einen Wert unterhalb 7 einstellt und die wirkstoffbeladenen Formkörper gefriertrocknet.

Pharmakadepots auf Basis Agarose und/oder Gelatine können in Analogie zu den Angaben in der DE-OS 26 57 370 hergestellt werden. Zweckmäßig inkorporiert man das Chemotherapeutikum in eine Lösung, die man aus physiologischer Kochsalzlösung und dem Polysaccharid erhalten hat, und der man vorteilhaft eine gewebediffusionsfördernde Substanz wie Hyaluronidase zusetzen kann, ferner auch knochenwachstumsfördernde Substanzen wie Calciumphosphat und/oder weitere Wirkstoffe. Derartige Präparate können bei einer Temperatur oberhalb der Körpertemperatur flüssig gehalten werden ; beim Abkühlen auf Körpertemperatur bilden sie stabile Gele.

Pharmakadepots auf Basis Fibrin liegen bevorzugt ebenfalls als Gele vor und können z.B. analog zu den Angaben in der DE-OS 32 06 725 hergestellt werden, zweckmäßig durch Mischen einer Fibrinogenlösung, einer Thrombinlösung und des Chemotherapeutikums. Das Fibrin wird dabei ausgefällt. Die Thrombinlösung enthält zweckmäßig zusätzlich Aprotinin und/oder ist mit Calcium-Ionen angereichert, z.B. in Form von $CaCl_2$. Es ist möglich, das Gel erst am Ort der Wahl, z.B. unmittelbar in der Knochenhöhle durch Zusatz der Thrombinlösung zu der Fibrinogenlösung zu bilden, wobei das Chemotherapeutikum, zweckmäßig in Form eines Salzes, entweder der Thrombinlösung oder der Fibrinogenlösung zugesetzt wird. Bevorzugt wird das Gel aber durch extrakorporale Mischung der Bestandteile hergestellt.

Es kann vorteilhaft sein, dem Pharmakadepot zusätzlich noch Wirkstoffe anderer Wirkrichtungen beizufügen, insbesondere Antibiotika und/oder Antiseptika zur Steigerung oder Ergänzung der Wirkung des Chemotherapeutikums. Anti biotika sollten möglichst sowohl gegen grampositve als auch gramnegative Erreger wirksam sein und sollten bei den Erregern keine oder nur eine verzögerte Resistenz hervorrufen. Beispielsweise als geeignet können Aminoglykosidantibiotika wie Amikacin, Butirosin, Didesoxykanamycin B (DKP), Fortimycin, Gentamycin, Kanamycin, Lividomycin, Neomycin, Netilmicin, Ribostamycin, Sagamycine, Seldomycine und deren Epimere, Sisomicin, Sorbistin, Tobramycin, Streptomycine, Lincomycine wie Clindamycin, Lincomycin und Rifamycine wie Rifampicin und Rifamycin genannt werden. Als Antiseptika kommen z.B. Bromchlorophen, Hexetidin, Buclosamid, Salicylsäure, Cernitrat, Chlorhexidin, 5-Chlor-8-hydroxy-chinolin, Kupfer-8-hydroxychinolat, Acridinorange, Undecensäure, Undecoyliumchlorid, Silbersalze wie Silbersulfadiazin, Mafenid, Nitrofurazol, Cloflucarban, Tribromsalan, Taurolin und Noxythiolin in Frage. Weiterhin kommen Kombinationen mit anderen Wirkstoffen, z.B. Zytostatika wie Methotrexat, Cisplatin, Adriamycin, Vinblastin und/oder Entzündungshemmern in Betracht. Auch diese zusätzlichen Wirkstoffe, deren Art und Menge sich nach der gewünschten zusätzlichen Wirkung richtet, können den anderen Materialien auf an sich übliche Weise, vorzugsweise in feinverteilter Pulverform, zugemischt werden, wobei auch hier gegebenenfalls eine Vormischung mit einzelnen der anderen Bestandteilen oder eine Einarbeitung in das Präpolymerisat erfolgen kann.

Die erfindungsgemäßen Pharmakadepots können in der Therapie insbesondere zur Bekämpfung von Infektionen verwendet werden.

Vor- und nachstehend sind alle Temperaturen in °C angegeben. Prozentangaben sind Gewichtsprozente, sofern sich aus dem Zusammenhang nichts anderes ergibt.

Beispiel 1

40 g eines sterilen, feinen Perlpolymerisats, bestehend aus Copolymerisat von Methylacrylat und Methylmethacrylat, das zusätzlich 0,5% Dibenzoylperoxid, Spuren Chlorophyll und 15% Zirkondioxid als Röntgenkontrastmittel enthält, werden mit 0,5 g Ciprofloxacin gut vermischt. Das erhaltene Pulver wird sodann mit 20 ml einer Flüssigkeit gemischt, die aus monomerem Methylmethacrylat besteht mit einem Zusatz von etwa 0,7% Dimethyl-p-toluidin und etwa 0,006% Hydrochinon. Aus der nach gründlicher Durchmischung entstehenden Paste werden Kügelchen mit einem Durchmesser von etwa 7 mm geformt. Nach etwa 6 Minuten sind die Partikeln erhärtet. Gegebenenfalls kann eine Sterilisation, z.B. durch Begasung mit Äthylenoxid, angeschlossen werden. Die Kügelchen können sodann zur Füllung osteomyelitischer Höhlen eingesetzt werden.

Beispiel 2

Analog Beispiel 1 werden Kügelchen mit einem Durchmesser von etwa 7-10 mm geformt. Der Zusatz an Ciprofloxacin beträgt 1 g auf 60 g Polymerisat. Noch vor der vollständigen Aushärtung werden die erhaltenen Partikeln mit einem Abstand von etwa 2 mm wie Perlen auf einen Faden (chirurgischer, hochlegierter Stahldraht) mit einem Durchmesser von etwa 0,1 mm aufgezogen. Man läßt die Kügelchen in diesem Zustand aushärten. Bei der Füllung osteomyelitischer Höhlen kann die "Perlschnur" auf jeder beliebigen Länge abgeschnitten werden.

Beispiel 3

Analog Beispiel 1 wird ein Granulat hergestellt von Partikeln mit einem mittleren Durchmesser von 6 bis 8 mm. Vor der Polymerisation werden 0,5 g Gentamycinsulfat und 1 g Ciprofloxacin zugefügt. Das erhaltene Produkt wird zur Füllung osteomyelitischer Höhlen verwendet.

Beispiel 4

Analog Beispiel 1 werden 40 g des Perlpolymerisats mit 0,8 g Ofloxacin versetzt. Die Polymerisation erfolgt nach Zugabe von 20 ml der das Monomere enthaltenden Flüssigkeit. Es werden Kügelchen mit einem Durchmesser von 7 bis 10 mm geformt. Diese können analog Beispiel 2 auf einen chirurgischen, biegsamen Draht aufgezogen werden, wodurch die Handhabung während der Operation erleichtert wird.

Beispiel 5

Man arbeitet analog Beispiel 1, setzt dem Pulvergemisch aber außerdem noch 0,4 g Glycin zu. Durch Vermischen des erhaltenen Pulvers mit dem monomeren Methylmethacrylat erhält man eine Paste, die von Hand oder mit Hilfe einer Spritze in eine Knochenhöhle eingebracht oder eingepreßt werden kann und die man dort aushärten läßt. In die in den Körper eingebrachte, noch nicht ausgehärtete Paste kann eine Prothese wie z.B. eine Endoprothese eingepreßt werden. Nach dem Aushärten des Kunststoffs ist die Prothese fest im Körper verankert.

Beispiel 6

36 g eines feinteiligen Copolymerisats aus Methylacrylat und Methylmethacrylat, das zusätzlich 0,5% Dibenzoylperoxid und Spuren Chlorophyll enthält, 4% mikronisiertes L-Arginin, 0,5 g Ciprofloxacin, 0,5 g Gentamycin und 20 ml Methylmethacrylat, das etwa 0,7% Dimethyl-p-toluidin und etwa 0,006% Hydrochinon enthält, werden gründlich vermischt. Aus der entstehenden Paste werden Kugeln, Stifte, Ovoide sowie auch größere Implantate wie Zylinder, Röhren, Platten, Folien und andere Teilchen beliebiger Form und Größe geformt, die nach wenigen Minuten ausgehärtet sind. Die Teilchen werden steril verpackt und können als lokale Wirkstoffdepots eingesetzt werden.

Beispiel 7

3 g Ciprofloxacin und 3 g penta-Calciumhydroxidtriphosphat werden gemischt und mit einer Tablettenpresse bei 20° und etwa 100 bar Preßdruck zu Tabletten gepreßt.

Beispiel 8

Man mischt 3 g Ciprofloxacin, 3 g Tricalciumphosphat und 0,3 g Calciumsulfat und preßt bei 20° und etwa 100 bar Preßdruck zu Tabletten.

Beispiel 9

Man mischt 3 g Ciprofloxacin, 3 g Hydroxylapatit und 1 g Proteinpulver und preßt bei 80° und etwa 100 bar Preßdruck zu Tabletten.

Beispiel 10

Ein Gemisch aus 475 g feingemahlenem Kollagen, 25 g eines Copolymeren aus 80 Molprozent L-Lactid und 20 Molprozent Glykolid mit einer reduzierten spezifischen Viskosität von 42 $cm^3/g$ und 10 g Ciprofloxacin wird homogenisiert und anschließend in einer geheizten Presse bei 135° und etwa 630 bar eine Minute in einer Metallnegativform zu Tabletten verpreßt (Durchmesser 1 cm, Höhe 2 mm).

Falls erwünscht, kann eine Sterilisation durch Begasung mit Ethylenoxid oder durch Bestrahlung angeschlossen werden. Die Tabletten können zur Infektionsprophylaxe bei Weichteilwunden oder Trümmerzonen bei offenen Knochenbrüchen oder auch zur Füllung osteomyelitischer Höhlen eingesetzt werden.

Beispiel 11

Ein Gemisch aus 375 g feingemahlenem Kollagen, 25 g eines Copolymeren aus 70 Molprozent L-Lactid und 30 Molprozent Glykolid mit einer reduzierten spezifischen Viskosität von 53 $cm^3/g$, 100 g gepulvertes Tricalciumphosphat und 10 g Norfloxacin wird homogenisiert und dann in einem Extruder bei einer Massetemperatur von etwa 145° zu einem Strang von 1,5 mm Durchmesser geformt, der anschließend in 1 mm Abstand zu einem Granulat zerkleinert wird.

Beispiel 12

Ein Gemisch aus 25 g Proteinpulver (hydrolysiertes wasserlösliches Kollagen ; mittleres Molekulargewicht ca. 3000), 10 g Ofloxacin und 100 g gepulvertem Tricalciumphosphat wird bei 90° und ca. 650 bar zwei Minuten gepreßt. Der Preßling wird dann gemahlen und das erhaltene Pulver mit 375 g Kollagen durchmischt. Das so erhaltende Gemisch wird eine Minute bei 85° und etwa 650 bar zu Kugeln (Durchmesser 6 mm) gepreßt.

Beispiel 13

200 ml einer wässerigen Lösung von 1,7 g Ciprofloxacin, die mit NaOH auf pH 9 eingestellt ist, wird mit 200 ml einer wässerigen Lösung von 1,7 g Pektinsäure-Natriumsalz (Veresterungsgrad ca. 8%), die ebenfalls mit NaOH auf pH 9 eingestellt ist, vermischt. In diese Mischung werden 10 g eines nach dem Verfahren des DBP 28 54 490 gewonnenen Kollagens in Form von quaderförmigen Teilchen mit den ungefähren Abmessungen 2x2x1 cm eingetragen. Durch mehrmaliges Evakuieren und anschließendes Belüften wird die Luft aus dem porösen Material entfernt. Unter Rühren stellt man den pH-Wert mit 1 n Schwefelsäure auf 6,5 ein. Das Gesamtvolumen beträgt danach etwa 500 ml. Die Kollagenstückchen werden entnommen, nach kurzem Abtropfen bei -20° eingefroren und danach lyophilisiert. Das so gewonnene Wirkstoffdepot kann durch Bestrahlung und z.B. durch Einsiegelung in Doppel-peel-Täschchen keimfrei verpackt werden.

Beispiel 14

Man suspendiert 20 g eines nach dem Verfahren gemäß der DE-PS 27 30 623 gewonnenen Kollagens in 1 l 0,5 molarer Essigsäure. Nach einer Äquilibrierungszeit von ca. 12 Std. wird der pH-Wert auf einen Wert zwischen 2,5 und 3,5 korrigiert. Die essigsaure Suspension wird homogenisiert, indem mittels eines Rührwerkes unter gleichzeitiger Kühlung die Länge der Kollagenfasern auf Werte unter 10 mm eingestellt wird. Nach dem Abpressen der so erhaltenen Kollagenmasse wird diese in Wasser aufgeschlämmt und erneut abgepreßt. Dieser Vorgang wird noch zweimal wiederholt. Das von überschüssiger Essigsäure befreite Kollagen wird dann unter leichtem Rühren in eine auf 35° temperierte wässerige Lösung von pH 9,5, die 1,2 g Ciprofloxacin und 1,2 g Natriumpektat in ca. 900 ml enthält, eingetragen. Nach einer Äquilibrierungszeit von 30 Minuten versetzt man mit 1-normaler Schwefelsäure bis pH 5-6 und ergänzt das Gesamtvolumen mit Wasser zu einem Liter. Die so erhaltene Masse wird in vorbereitete Gießformen gegossen, bei -20 bis 40° schockgefroren und anschließend gefriergetrocknet. Man erhält ein flächiges Wirkstoff-Depot vliesartiger Beschaffenheit mit einem Gehalt von 4% Ciprofloxacin, welches entsprechend der vorgegebenen Schichthöhe des KollagenBreis eine Dicke von 2 mm bis zu etwa 10 mm besitzt. Das Flächengewicht beträgt dann etwa 40 g pro $m^2$, bzw. 200 g pro $m^2$. Die Wasseraufnahmekapazität des Wirkstoff-Depots liegt bei etwa 2000% bezogen auf Trockenmasse.

Beispiel 15

Man kocht 2 g Agarose-Pulver mit 100 ml physiologischer Kochsalzlösung 30 Minuten und kühlt auf 50° ab. Von der erhaltenen sterilisierten Lösung werden 2 ml mit 150 I.E. Hyaluronidase und 10 mg Ciprofloxacin vermischt und thermostatisiert bis zur Verwendung zum Einsetzen von enossalen Dental-Halbimplantaten bei 50° gehalten.

Beispiel 16

2 ml der nach Beispiel 15 erhaltenen Lösung werden zusätzlich mit 20 mg Apatit-Pulver vermischt. Das so erhaltene Produkt eignet sich besonders zum Einsetzen von Implantaten.

Beispiel 17

2 ml der nach Beispiel 15 erhaltenen Lösung werden mit 0,7 g denaturiertem Knochenmehl vermischt. Das so erhaltene Gemisch dient besonders zum Ausfüllen von Hohlräumen, die bei Knochenoperationen entstehen.

Beispiel 18

Man löst 4 NIH-Einheiten Thrombin (Handelspräparat) in 1 ml Aprotinin-Calciumchlorid-Lösung (Handelspräparat ; 3000 KIE/ml Aprotinin in 40 mMol/l $CaCl_2$) erwärmt die Lösung auf 37°, gibt 20 mg Ciprofloxacin hinzu und mischt mit der gleichen Menge vorher auf 37° erwärmtem "Fibrinkleber" (Handelsprätaparat ; hergestellt durch Kältepräzipitation aus humanem Spenderplasma ; bei -18° oder kälter gelagert ; 1 ml der Lösung enthält durchschnittlich 90 mg thrombinfällbares Protein, Gesamteiweißgehalt der Lösung etwa 10% ; etwa 20-30 Minuten vor der geplanten Verwendung aufgetaut). Man läßt das Gemisch in Zylindern aus rostfreiem Stahl (Innendurchmesser 6 mm, Höhe 10 mm) erstarren (1 ml für 3 Zylinder). Die gebildeten Gel-Zylinder werden anschließend aus den Formen ausgestoßen.

## Ansprüche

1. Implantierbares Pharmakadepot, im wesentlichen bestehend aus physiologisch verträglichen Trägermaterialien der Gruppe Polyacrylate und/oder Polymethacrylate, TricalciumPhosphat, Hydroxylapatit, Kollagen, Agarose, Gelatine und/ oder Fibrin und mindestens einem verzögert freizusetzenden Wirkstoff, dadurch gekennzeichnet, daß es als Wirkstoff ein Chemotherapeutikum des Gyrasehemmertyps enthält.

## Claims

1. Implantable medicament depot essentially consisting of physiologically acceptable excipients of the group comprising polyacrylates and/or polymethacrylates, tricalcium phosphate, hydroxyapatite, collagen, agarose, gelatine and/or fibrin and at least one active compound for delayed release, characterized in that it contains a chemotherapeutic of the gyrase inhibitor type as the active compound.

## Revendications

1. Composition pharmaceutique implantable à effet dépôt, constituée essentiellement par des matières de support physiologiquement acceptables choisies parmi le groupe comprenant : les polyacrylates et/ou les polyméthacrylates, le phosphate tricalcique, l'apatite hydroxylée, le collagène, l'agarose, les gélatines et/ou la fibrine, et par au moins une substance active à libération retardée, **caractérisée en ce qu'**elle contient, comme substance active, un agent chimiothérapeutique du type inhibiteur de gyrase.